# EUROPÄISCHE PATENTSCHRIFT

(11) **EP 3 954 328 B1**
(45) Veröffentlichungstag und Bekanntmachung des Hinweises auf die Patenterteilung: **10.06.2026**
(21) Anmeldenummer: 20190807.6
(22) Anmeldetag: 13.08.2020
(51) Int. Cl.: A61F 2/18

(54) **GEHÖRKNÖCHELCHENPROTHESE**
OSSICULAR PROSTHESIS
PROTHÈSE D'OSSELETS DE L'OREILLE

(43) Veröffentlichungstag der Anmeldung: 16.02.2022
(73) Patentinhaber: MED-EL Elektromedizinische Geräte GmbH, 6020 Innsbruck (AT)
(72) Erfinder: MAIR, Lukas, 6130 Schwaz (AT); STEINHARDT, Uwe, 72145 Hirrlingen (DE); KUEN, Clemens, 6460 Imst (AT)
(74) Vertreter: Witte, Weller & Partner Patentanwälte mbB

(56) Entgegenhaltungen:
- EP-B1- 3 512 463
- US-A1- 2002 095 063
- US-B1- 6 325 755

## Beschreibung

Die Erfindung betrifft eine Gehörknöchelchenprothese mit den Merkmalen des Oberbegriffs des Anspruchs 1

Gehörknöchelchenprothesen dienen als Ersatz eines oder mehrerer Gehörknöchelchen im menschlichen Mittelohr und übertragen anstatt des oder der ersetzten Gehörknöchelchen durch Schallwellen erzeugte Schwingungen des Trommelfells auf das ovale Fenster des Innenohrs. Eine Länge einer Gehörknöchelchenprothese muss individuell an einen zu überbrückenden Abstand vom Trommelfell zu dem anzukoppelnden Gehörknöchelchen, beispielsweise dem Steigbügel des jeweiligen Patienten angepasst werden.

Das europäische Patent EP 3 512 463 B1 offenbart eine einstückige Gehörknöchelchenprothese, die aus einem Blech heraus getrennt und zu der Gehörknöchelchenprothese gebogen ist. Die bekannte Gehörknöchelchenprothese weist ein ebenflächiges Kopfelement und zwei von dem Kopfelement abstehenden, U-förmige Klemmen auf. Das Kopfelement weist ein ebenes, rechteckiges Mittelteil mit spiegelbildlich an gegenüberliegenden Seiten des Mittelteils angeordneten, Ω-förmigen Bügeln in einer Ebene mit dem Mittelteil, deren Füße einstückig in das Mittelteil übergehen, auf. Das Kopfelement ist zu einer Anlage an einer Innenseite des Trommelfells vorgesehen. Zwischen den Ω-förmigen Bügeln stehen die beiden U-förmigen Klemmen nahezu rechtwinklig zum Kopfelement zur selben Seite ab. In Seitenansicht sind die beiden Ω -förmigen Klemmen deckungsgleich. Ihre Joche sind über kurze, nahezu rechtwinklig in die Ebene des Kopfelements umgebogene Stege einstückig mit dem Mittelteil des Kopfelements verbunden. Schenkel der U-förmigen Klemmen bilden Klemmbacken zu einem klemmenden Aufsetzen auf ein Gehörknöchelchen. Die die Klemmbacken bildenden Schenkel der U-förmigen Klemmen sind zick-zack-förmig. Ein Nachteil der bekannten Gehörknöchelchenprothese ist, dass die zick-zack-förmigen Klemmbacken beim Aufsetzen auf das Gehörknöchelchen eine Kraft auf das Gehörknöchelchen ausüben.

Die Patentanmeldung US 2002/0 095 063 A1 offenbart einen Federclip, der an einem akustischen Signalwandler befestigt und auf ein Gehörknöchelchen geklipst wird, um mechanische Schwingungen, die der Signalwandler erzeugt, auf das Gehörknöchelchen zu übertragen.

Aufgabe der Erfindung ist eine Gehörknöchelchenprothese der vorstehend erläuterten Art anzugeben, die sich leichter auf ein Gehörknöchelchen aufsetzen lässt.

Diese Aufgabe wird erfindungsgemäß durch die Merkmale des Anspruchs 1 gelöst. Die erfindungsgemäße Gehörknöchelchenprothese ist zangenartig ausgebildet. Sie weist ein zwei- oder mehrteiliges Kopfelement mit mindestens zwei Kopfteilen und eine oder mehrere Klemmen mit Klemmbacken zu einem klemmenden Aufsetzen auf ein Gehörknöchelchen, insbesondere auf einen stab- oder stangenförmigen Abschnitt eines Gehörknöchelchens, auf. Das Kopfelement ist zu einer Anordnung an einer Innenseite eines Trommelfells so vorgesehen, dass durch Schallwellen erzeugte Schwingungen des Trommelfells auf die Gehörknöchelchenprothese übertragen werden. Als Trommelfell ist in diesem Zusammenhang sowohl ein vorhandenes, teilweise vorhandenes als auch ein künstlich vollständiges oder teilweise rekonstruiertes Trommelfell sowie ein teilweise vorhandenes und im Übrigen rekonstruiertes Trommelfell zu verstehen. Die Rekonstruktion umfasst jegliche Form der Herstellung einer schwingfähigen Membran, beispielsweise mit Körpegewebe oder einer künstlich angelegten Schwingungsmembran zur Wandlung von Schall in mechanische Schwingungen. Mit umfasst ist eine Anordnung oder Befestigung des Kopfelements an einem Gehörknöchelchen anstatt am Trommelfell.

Zwei Kopfteile des Kopfelements sind gegeneinander beweglich, insbesondere lässt sich ihr Abstand voneinander vergrößern und/oder verkleinern. Die Klemmbacken sind ebenfalls gegeneinander beweglich, insbesondere sind sie zueinander schwenkbar, so dass sich ihr Abstand voneinander ebenfalls vergrößern und/oder verkleinern lässt. Eines der Kopfteile ist mit einer Klemmbacke und ein anderes Kopfteil ist mit der anderen Klemmbacke starr oder jedenfalls so steif verbunden, dass sich durch eine Bewegung der Kopfteile gegeneinander, insbesondere durch eine Änderung ihres Abstands voneinander vergleichbar den Griffen einer Zange der Abstand der Klemmbacken voneinander ändern lässt. Insbesondere vergrößert sich der Abstand zwischen den Klemmbacken, wenn die Kopfteile einander genähert werden und umgekehrt. Es sind auch Ausführungen der erfindungsgemäßen Gehörknöchelchenprothese denkbar, bei der sich der Abstand der Klemmbacken voneinander vergrößert, wenn der Abstand der Kopfteile des Kopfelements voneinander vergrößert wird. Ein Chirurg kann die Klemme der Gehörknöchelchenprothese auf ein Gehörknöchelchen aufsetzen, indem er das Kopfelement der Gehörknöchelchenprothese in einer Ausgangslage mit beispielsweise einer Pinzette greift, die er an den beiden Kopfteilen ansetzt. Die Pinzette setzt der Chirurg von einer der Klemme gegenüberliegenden Seite an dem Kopfelement der Gehörknöchelchenprothese an. Indem er die Pinzette schließt, drückt er die beiden Kopfteile zusammen, womit sich ihr Abstand voneinander verkleinert. Die so an den Kopfteilen zusammengedrückte Gehörknöchelchenprothese befindet sich in einer Ankopplungslage, in der sich die Klemme öffnet, das heißt die beiden Klemmbacken entfernen sich voneinander, so dass der Chirurg die Klemme beziehungsweise die Klemmbacken der Gehörknöchelchenprothese in besonders vorteilhafter Weise auf das Gehörknöchelchen aufsetzen kann, ohne eine Kraft auf das Gehörknöchelchen auszuüben. Damit wird eine Beschädigung der empfindlichen Gehörknöchelchen vermieden. Öffnet der Chirurg die Pinzette und lässt damit Gehörknöchelchenprothese langsam los, federn die Klemmbacken der Klemme zusammen und halten die Gehörknöchelchenprothese klemmend an dem Gehörknöchelchen. Insbesondere federn die Klemmbacken elastisch zurück in die Ausgangslage beziehungsweise in Anlage an das Gehörknöchelchen, wenn die Kopfteile losgelassen werden. Sie liegen mit einer Vorspannung an dem Gehörknöchelchen an und halten dadurch die Gehörknöchelchenprothese an dem Gehörknöchelchen.

Die Pinzette löst sich erst dann von der Gehörknöchelchenprothese, wenn keine Kraft auf die Pinzettenarme infolge des Entgegenwirkens zur Vorspannung von der Gehörknöchelchenprothese wirkt, was genau dann der Fall ist, wenn die gesamte Vorspannung der Gehörknöchelchenprothese auf die Klemmbacken wirkt. Damit wird sichergestellt, dass erst wenn die Gehörknöchelchenprothese sicher auf dem Gehörknöchelchen platziert und fixiert ist, die Pinzette sich von der Gehörknöchelchenprothese löst. Das erleichtert beispielsweise die Handhabung und Platzierung während des chirurgischen Eingriffs erheblich und verhindert unter anderem ein unbeabsichtigtes oder vorzeitiges Lösen der Gehörknöchelchenprothese von der Pinzette, beispielsweise vor exakter Positionierung, Ausrichtung und Fixierung. Zudem kann eine Pinzette verwendet werden, die es nur erlaubt eine kleine Haltekraft auf die Gehörknöchelchenprothese auszuüben und damit plastische Verformungen der Gehörknöchelchenprothese zu verhindern.

Ein zusätzlicher Vorteil der Erfindung ist, dass sich die Klemme der Gehörknöchelchenprothese auf ein Gehörknöchelchen aufsetzen lässt ohne während der Platzierung eine Kraft auf das Gehörknöchelchen auszuüben.

Eine Ausgestaltung der Erfindung sieht einen verformbaren Steg vor, der die Klemmbacken abstandsveränderlich verbindet. Der Steg bildet eine Art Gelenk; durch eine Verformung des Stegs lässt sich der Abstand zwischen den Klemmbacken verändern. Insbesondere wird der Steg bei der Änderung des Abstands der Klemmbacken voneinander gebogen. Diese Ausgestaltung der Erfindung ermöglicht eine einstückige Ausführung der Klemme und/oder der Gehörknöchelchenprothese.

Ein Abstand der Klemmbacken voneinander ist zu einer Anpassung an eine Dicke des Gehörknöchelchens bei Ausführungen der Erfindung durch plastische Verformung insbesondere des die Klemmbacken verbindenden, verformbaren Stegs und/oder der Klemmbacken einstellbar. Der Abstand der Klemmbacken wird kleiner als die Dicke des Gehörknöchelchen eingestellt, so dass die Klemmbacken elastisch mit der Vorspannung an dem Gehörknöchelchen anliegen, wenn die Klemme der Gehörknöchelchenprothese auf das Gehörknöchelchen aufgesetzt ist.

Die Klemme kann durch einen Schlitz in einem die Klemme bildenden Bereich der Gehörknöchelchenprothese gebildet sein. Bereiche beiderseits des Schlitzes bilden die Klemmbacken, deren Abstand voneinander durch den Schlitz veränderbar ist. Eine Ausgestaltung der Erfindung sieht eine U-förmige Klemme vor, wobei Schenkel des "U" die Klemmbacken bilden.

Für einen besseren Halt an dem Gehörknöchelchen sieht eine Ausgestaltung der Erfindung mindestens zwei Klemmen vor. Insbesondere sind die Klemmen deckungsgleich. Allerdings genügt es, wenn die Klemmen in einem Abstand voneinander so angeordnet sind, dass sie auf das gleiche Gehörknöchelchen aufsetzbar sind.

Eine bevorzugte Ausgestaltung sieht vor, dass einander zugewandte Ränder oder Flächen der Klemmbacken eine Wellung, Zacken oder Zähne aufweisen. Dadurch ändert sich ein lichter Abstand der einander zugewandten Ränder oder Flächen der Klemmbacken, der Abstand vergrößert und verkleinert sich abwechselnd in einer Längsrichtung der Klemmbacken und die Klemme der Gehörknöchelchenprothese lässt sich so auf beispielsweise einen stab- oder stangenförmigen Abschnitt des Gehörknöchelchens aufsetzen, so dass sich der stab- oder stangenförmigen Abschnitt in einander gegenüberliegenden Wellentälern oder in einander gegenüberliegenden Zwischenräumen zwischen Zacken oder Zähnen der einander zugewandten Ränder oder Flächen der Klemmbacken befinden. Dadurch ist ein Halt der Gehörknöchelchenprothese in der Längsrichtung ihrer Klemmbacken auf dem Gehörknöchelchen verbessert und es lässt sich ein Abstand des Kopfelements von dem Gehörknöchelchen in Schritten im Abstand der Wellentäler oder der Zwischenräume zwischen den Zacken oder Zähnen einstellen. Dadurch ist problemlos eine Anpassung der erfindungsgemäßen Gehörknöchelchenprothese an einen Abstand des Trommelfells von dem Gehörknöchelchen möglich.

Eine Ausgestaltung der Erfindung sieht vor, dass sich ein Abstand der Klemmbacken voneinander von einem freien Ende zu einem geschlossenen Ende der Klemme vergrößert. Dadurch wird am freien Ende eine Öffnung zwischen den Klemmbacken gebildet. Bei gewellten oder gezackten einander zugewandten Rändern oder Flächen der Klemmbacken vergrößern sich Abstände einander gegenüberliegender Wellenberge und/oder Wellentäler beziehungsweise einander gegenüberliegender Zacken oder Zähne, beziehungsweise allgemein Berge und Täler und/oder Zwischenräume zwischen den Zacken oder Zähnen von den freien Enden der Klemmbacken zum geschlossenen Ende der Klemme. Dadurch ist eine zumindest näherungsweise gleiche Klemmkraft möglich unabhängig davon, wie weit das Gehörknöchelchen vom freien Ende entlang der Längsrichtung hin zum geschlossenen Ende in der Klemme angeordnet ist.

Sämtliche in der Beschreibung genannte und/oder der Zeichnung dargestellte Merkmale können einzeln für sich oder in jeder beliebigen Kombination bei Ausführungen der Erfindung verwirklicht sein. Ausführungen der Erfindung, die nicht alle, sondern nur einen Teil der Merkmale eines Anspruchs, auch des unabhängigen Anspruchs, aufweisen, sind möglich.

Die Erfindung wird nachfolgend anhand eines in der Zeichnung dargestellten Ausführungsbeispiels näher erläutert. Es zeigen:
- Figur 1: eine Gehörknöchelchenprothese gemäß der Erfindung in perspektivischer Darstellung;
- Figur 2: eine Seitenansicht der Gehörknöchelchenprothese aus Figur 1 in einer Ausgangslage;
- Figur 3: die Gehörknöchelchenprothese aus Figur 2 mit einer aufgeweiteten Klemme in einer Ankopplungslage;
- Figur 4: die Gehörknöchelchenprothese aus Figur 2 mit verengter Klemme ; und
- Figur 5: die Gehörknöchelchenprothese aus Figur 2 mit bemaßten Wellentälern.

Die in der Zeichnung dargestellte, erfindungsgemäße Gehörknöchelchenprothese 1 ist zum Ersatz eines oder mehrerer Gehörknöchelchen in einem Mittelohr eines Menschen vorgesehen. Sie weist ein Kopfelement 2, das zu einer Anordnung an einer Innenseite eines Trommelfells des Ohrs, und zwei Klemmen 3, die zu einem klemmenden Aufsetzen auf ein Gehörknöchelchen zu einer Befestigung der Gehörknöchelchenprothese 1 an dem Gehörknöchelchen vorgesehen ist, auf. Insbesondere werden die Klemmen 3 auf einen Schenkel eines Steigbügels im Mittelohr eines Menschen aufgesetzt. Sie lassen sich allerdings ebenso auf einen langen Ambossschenkel eines Amboss oder einen Hammergriff eines Hammers des Ohrs aufsetzen oder an anderer Stelle befestigen. Die Gehörknöchelchenprothese 1 überträgt durch Schallwellen erzeugte Schwingungen des Trommelfells auf den Steigbügel oder das Element des Ohrs, an dem die Klemmen 3 befestigt sind. Als Trommelfell ist in diesem Zusammenhang sowohl ein vorhandenes, teilweise vorhandenes als auch ein künstlich vollständig oder teilweise rekonstruiertes Trommelfell sowie ein teilweise vorhandenes und im Übrigen rekonstruiertes Trommelfell zu verstehen. Die Rekonstruktion umfasst jegliche Form der Herstellung einer schwingfähigen Membran, beispielsweise mit Körpergewebe oder einer künstlich angelegten Schwingungsmembran zu einer Wandlung von Schall in mechanische Schwingungen.

Das Kopfelement 2 weist zwei gegeneinander bewegliche, nämlich in ihrem Abstand voneinander veränderliche Kopfteile 4 auf. In Ansicht auf das Kopfelement 2, das heißt in einer Stirnansicht der Gehörknöchelchenprothese 1, weisen die Kopfteile 4 Ω-förmige Bügel 5 auf, die spiegelsymmetrisch zu einer Mittelebene der Gehörknöchelchenprothese 1 angeordnet sind, wobei "Füße" der Ω-förmigen Bügel 5 einander zugewandt sind. In ihrer Mittelebene weisen die Ω-förmigen Bügel 5 pfeilförmige Stege 6 zu ihrer Aussteifung auf. Die Mittelebene der Gehörknöchelchenprothese 1 und die Mittelebene der Ω-förmigen Bügel 5 stehen senkrecht zueinander. Die Form der Kopfteile 4 ist nicht zwingend für die Erfindung; ebenso wenig muss das Kopfelement 2 ebenflächig sein, sondern es kann beispielsweise auch gewölbt und/oder voluminös sein (nicht dargestellt).

Die "Füße" der beiden Ω-förmigen Bügel 5 der Kopfteile 4 gehen einstückig in Stege 7 über, die parallel zu der Mittelebene der Gehörknöchelchenprothese 1 verlaufen. An ihren Enden sind die beiden Stege 7 rechtwinklig oder jedenfalls ungefähr rechtwinklig zur gleichen Seite des Kopfelements 2 in vier Schäfte 8 umgebogen, die parallel zu der Mittelebene der Gehörknöchelchenprothese 1 verlaufen.

An ihren dem Kopfelement 2 fernen Enden gehen die Schäfte 8 - im Ausführungsbeispiel einstückig - in Klemmbacken 9 über, die in Seitenansicht der Gehörknöchelchenprothese 1 sich bogenförmig von dem Kopfelement 2 weg erstrecken. Konvexe Außenseiten oder Außenränder der Klemmbacken 9 sind einander abgewandt und konkave Innenseiten oder Innenränder der Klemmbacken 9 sind einander zugewandt. Als Schäfte 8 werden hier Abschnitte zwischen den Stegen 7 und den Klemmbacken 9 bezeichnet.

Die Gehörknöchelchenprothese 1 weist vier Klemmbacken 9 auf, die einander paarweise zugeordnet sind, wobei jeweils zwei einander zugeordnete Klemmbacken 9 eine Klemme 3 der Gehörknöchelchenprothese 1 bilden. Einander zugeordnet sind jeweils die beiden einander bezüglich der Mittelebene der Gehörknöchelchenprothese 1 spiegelbildlich gegenüber angeordneten Klemmbacken 9. Die Gehörknöchelchenprothese 1 weist zwei Klemmen 3 in zwei zueinander parallelen und zur Mittelebene der Gehörknöchelchenprothese 1 senkrechten Ebenen auf. In Seitenansicht sind die Klemmen 3 deckungsgleich. Möglich ist auch, dass sich die Klemmen 3 in zueinander schiefen Ebenen oder auch gekrümmten Flächen befinden und/oder dass die Klemmen 3 nicht deckungsgleich sind und/oder dass die Klemmbacken 9 nicht spiegelsymmetrisch sind (nicht dargestellt). Die Gehörknöchelchenprothese 1 weist vorzugsweise zwei Klemmen 3 auf, nicht ausgeschlossen sind allerdings nur eine Klemme 3 oder mehr als zwei Klemmen 3 (nicht dargestellt). Die Klemmen 3 können auch als U-förmigen aufgefasst werden, weil Schenkel der "U's" die Klemmbacken 9 bilden.

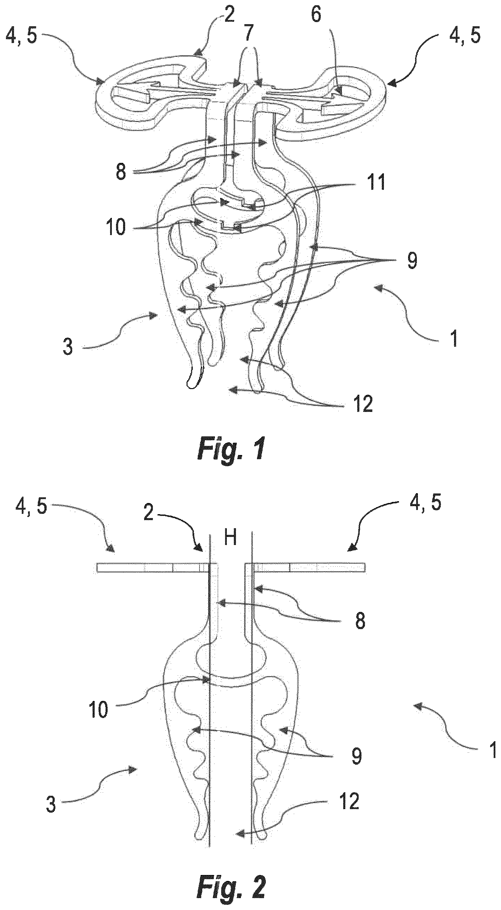

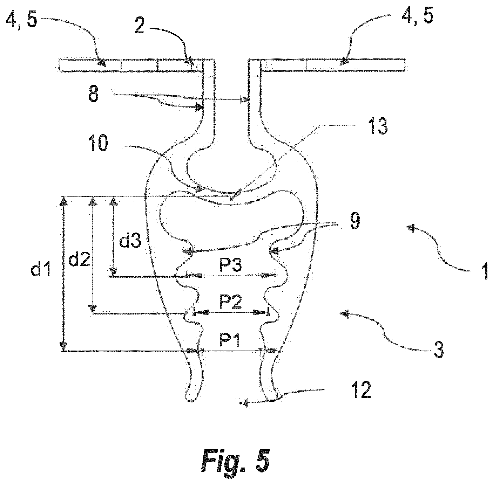

Die einander zugewandten Innenseiten oder Innenränder der Klemmbacken 9 weisen eine Wellung auf, wobei im Ausführungsbeispiel die Wellen verschieden hoch sind: wie die beiden parallelen geraden Hilfslinien H in Figur 2 und die Bemaßungen in Figur 5 veranschaulichen, vergrößert sich ein Abstand sowohl von Wellenbergen als auch von Wellentälern der Wellungen der Innenseiten oder Innenränder der Klemmbacken 9 von freien Enden der Klemmbacken 9, das heißt von einer Öffnung 13 der Klemmen 3 in Richtung geschlossener Enden der Klemmen 3, das heißt in Richtung des Kopfelements 2. Die Figuren 2 und 5 sowie Figur 1 zeigen die Gehörknöchelchenprothese 1 in der bevorzugten Ausgangslage, wobei die Kopfteile 4 ebenflächig angeordnet sind. Andere Positionen der Kopfteile 4 zueinander sind ebenfalls denkbar, wobei für die Ausgangslage kennzeichnend ist, dass keine Vorspannung durch elastisch verformte Stege 10 auf die Klemmbacken 9 beziehungsweise Kopfteile 4 wirkt.

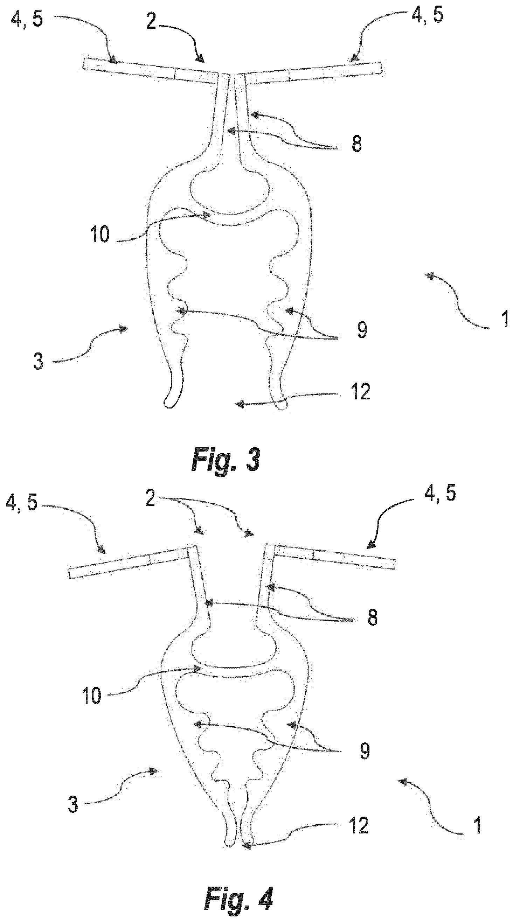

Die Klemmbacken 9 sind nahe ihren Übergängen in die Schäfte 8 durch Stege 10 einstückig miteinander verbunden. Im Ausführungsbeispiel verlaufen die Stege 10 bogenförmig, was allerdings nicht zwingend für die Erfindung ist. Die die Klemmbacken 9 verbindenden Stege 10 sind so schmal, dass sie verformbar sind, im Ausführungsbeispiel sind sie insbesondere biegbar. Die Stege 10 bilden eine Art Gelenke, die die Klemmbacken 9 schwenkbar miteinander verbinden. Werden - wie in Figur 3 gezeichnet - die Kopfteile 4 aufeinander genähert, schwenken die Klemmbacken 9 auseinander, die Klemmen 3 werden geöffnet. Werden umgekehrt die Kopfteile 4 voneinander entfernt, schwenken die Klemmbacken 9 zusammen und die Klemmen 3 werden geschlossen wie es Figur 4 zeigt. Das bedeutet, dass vergleichbar einer Zange die Klemmen 3 geschlossen und geöffnet werden können, wobei die Kopfteile 4 als "Zangengriffe" fungieren. Umgekehrt wie bei vielen Zangen werden die Klemmen 3 der Gehörknöchelchenprothese 1 geöffnet, indem die Kopfteile 4 einander genähert werden. Die Erfindung schließt Ausführungen der Gehörknöchelchenprothese 1, bei der die Klemmen 3 durch Nähern der Kopfteile 4 geschlossen werden, nicht aus.

Abweichend von den Figuren 2 bis 4 weisen die Stege 10 in Figur 1 eine Schmalstelle 11 auf, die eine Verformbarkeit der Stege 10 erleichtern. Die Stege 10 sind die einzigen Verbindungen der Klemmbacken 9 der Klemmen 3 und über die Schäfte 8 auch der Kopfteile 4 mit den Ω-förmigen Bügeln 5 des Kopfelements 2 der erfindungsgemäßen Gehörknöchelchenprothese 1. Ansonsten sind die Kopfteile 4 und die Klemmbacken 9 getrennt voneinander, sie sind spiegelbildlich zur Mittelebene der Gehörknöchelchenprothese 1 beiderseits der Mittelebene in einem Abstand voneinander angeordnet.

Die Stege 7 an den "Füßen" der Ω-förmigen Bügel 5 und die Schäfte 8 sind starr oder jedenfalls steifer als die Stege 10, die als Gelenke die Klemmbacken 9 schwenkbar verbinden. Die Schäfte 8 und die Stege 7 sind jedenfalls so steif, dass sich über sie durch zusammen- oder auseinander Bewegen der Kopfteile 4 die Klemmbacken 9 auseinander oder zusammen bewegen und damit die Klemmen 3 öffnen und schließen lassen, selbst wenn sich die Stege 7 und die Schäfte 8 dabei geringfügig elastisch verformen.

Um die erfindungsgemäße Gehörknöchelchenprothese 1 an einem Gehörknöchelchen, beispielsweise an einem Steigbügel in einem Mittelohr und dort insbesondere an einem Schenkel des Steigbügels zu befestigen ohne eine Kraft außer einer Klemmkraft auf das Gehörknöchelchen auszuüben, werden die Klemmen 3 der Gehörknöchelchenprothese 1 geöffnet, das heißt die Klemmbacken 9 auseinander geschwenkt, indem die Kopfteile 4 aufeinander zu bewegt werden, wie es Figur 3 zeigt. Dazu kann ein Chirurg die Gehörknöchelchenprothese 1 von einer den Klemmen 3 gegenüberliegenden Seite des Kopfelements 2 aus mit beispielsweise einer Pinzette an den Kopfteilen 4 greifen und die Kopfteile 4 zusammendrücken. Die dadurch geöffneten Klemmen 3 lassen sich auf das Gehörknöchelchen aufsetzen ohne eine Kraft auf das Gehörknöchelchen auszuüben. Werden die Kopfteile 4 jetzt losgelassen, schließt eine Federkraft der beim Öffnen der Klemmen 3 elastisch verformten Stege 10 die Klemmen 3 und drückt die Klemmbacken 9 von zwei entgegengesetzten Seiten gegen das Gehörknöchelchen, so dass die Gehörknöchelchenprothese 1 klemmend an dem Gehörknöchelchen befestigt ist.

Zu einer Anpassung an unterschiedlich dicke Gehörknöchelchen kann die Gehörknöchelchenprothese 1 vor dem Aufsetzen auf das Gehörknöchelchen plastisch verformt werden. Insbesondere werden die die Klemmbacken 9 verbindenden Stege 10 plastisch verformt. Figur 4 zeigt beispielsweise ein plastisches Zusammenbiegen der Klemmbacken 9 zu einer Anpassung der Klemmen 3 an ein dünneres Gehörknöchelchen, beispielsweise an einen Schenkel eines Steigbügels mit einem kleinen Durchmesser.

Indem die Klemmen 3 unterschiedlich weit auf das Gehörknöchelchen aufgesetzt werden, so dass sich das Gehörknöchelchen in einem Wellental der Klemmbacken 9 befindet, lässt sich ein Abstand des Kopfelements 2 von dem Gehörknöchelchen einstellen und an einen Abstand des Trommelfells von dem Gehörknöchelchen anpassen.

Die unterschiedlichen Abstände der Wellenberge und Wellentäler der Wellungen der Innenseiten oder Innenränder der Klemmbacken 9 sorgen dafür, dass die Klemmkräfte der Klemmbacken 9 an dem Gehörknöchelchen nicht zu hoch sind, wenn das Gehörknöchelchen nahe dem geschlossenen Ende der Klemmen 3 festgeklemmt wird, wo aufgrund der kurzen Abstände von beziehungsweise der kurzen Hebelarme zu den Stegen 10 höhere Kräfte wirken als nahe dem offenen Ende der Klemmen 3, wo die Abstände von den Stegen 10 beziehungsweise die Hebelarme länger sind.

Diese vorteilhafte Weiterbildung der Erfindung ist in Figur 5 durch die Bemaßung der Gehörknöchelchenprothese 1 in ihrer Ausgangslage dargestellt. Dabei bezeichnet d1, d2 und d3 den jeweiligen Abstand der Wellentäler vom geschlossenen Ende der Klemmen 3, gemessen von den Stegen 10. Die jeweiligen Abstände zwischen gegenüberliegenden Wellentälern der Wellungen der Innenseiten oder Innenränder der Klemmbacken 9 sind mit P1, P2 und P3 bezeichnet. Dabei ist im Wellental bei P3 der Abstand am größten, im Wellental bei P1 am geringsten und im Wellental bei P2 ist der Abstand geringer als bei P3 aber größer als bei P1. In derselben Weise trifft das für die Abstände gegenüberliegender Wellenberge zu, deren Abstände in Richtung zum geschlossenen Ende der Klemmen 3 zunehmen.

In einer weiteren vorteilhaften Weiterbildung der erfindungsgemäßen Gehörknöchelchenprothese 1 sind die Produkte gebildet aus den Abständen gegenüberliegender Wellentäler und dem jeweiligen Abstand dieser Wellentäler vom durch den Steg 10 vorgegebenen Drehpunkt 13 am geschlossenen Ende gleich. Dadurch ist unabhängig davon in welchem Wellental entlang der Längsrichtung an den Klemmbacken 9 das Gehörknöchelchen fixiert ist, die Haltekraft, die infolge der Vorspannung auf das Gehörknöchelchen wirkt, immer dieselbe. Veranschaulicht am Beispiel der Figur 5 entspricht das dem folgenden Zusammenhang: *P*1 · *d*1 *= P*2 · *d*2 *= P*3 · *d*3*,* wobei die Abstände d1, d2 und d3 gemessen sind von einem durch den Steg 10 vorgegebenen Drehpunkt 13, um den die Klemmbacken 9 bei elastischer Verformung des Stegs 10 schwenken. Wie zuvor ausgeführt, ist diese Ausführungsform der Erfindung keinesfalls auf einander zugewandte Innenflächen oder Innenränder der Klemmbacken 9, die eine Wellung aufweisen, beschränkt. Als Zacken oder Zähne ausgebildete Innenflächen oder Innenränder der Klemmbacken 9 sind ebenfalls geeignet. In der Tat, jegliche Formung der Innenseiten oder Innenränder der Klemmbacken 9 die Täler und Berge aufweist ist geeignet.

In einer weiteren vorteilhaften Weiterbildung ist die erfindungsgemäßen Gehörknöchelchenprothese 1 derart ausgebildet, dass sich in der Ausgangslage der Gehörknöchelchenprothese 1 ein Abstand gegenüberliegender Berge an den Innenflächen oder Innenrändern der Klemmbacken 9 voneinander von einem freien Ende zu einem geschlossenen Ende der Klemme 3 vergrößert. Zusätzlich oder alternativ kann die erfindungsgemäße Gehörknöchelchenprothese 1 so ausgebildet sein, dass sich in der Ankopplungslage der Gehörknöchelchenprothese 1 ein Abstand gegenüberliegender Berge voneinander von einem freien Ende zu einem geschlossenen Ende der Klemme 3 verkleinert. Das hat den weiteren Vorteil, dass wenn sich die Gehörknöchelchenprothese 1 zum Aufbringen auf ein Gehörknöchelchen in Ankopplungslage befindet, das Gehörknöchelchen nicht beschädigt wird wenn es zum Aufbringen vom freien Ende zum geschlossenen Ende der Klemme 3 entlang der Längsrichtung der Klemmbacken 9 geführt wird, weil es nicht an Bergen vorbei streift unabhängig davon, wie weit bzw. wo in Längsrichtung auf den Klemmbacken 9 in der Klemme 3 das Gehörknöchelchen fixiert wird.

Die erfindungsgemäße Gehörknöchelchenprothese 1 ist aus einem Stück hergestellt, sie ist durch Laserschneiden aus einem Blech heraus getrennt und zu der Gehörknöchelchenprothese 1 gebogen. Andere Herstellungsarten und/oder eine Herstellung aus mehreren Teilen sind möglich.

## Patentansprüche

1. Gehörknöchelchenprothese, mit einem Kopfelement (2), das zu einer Anordnung an einem Trommelfell vorgesehen ist, und mit einer zwei Klemmbacken (9) aufweisenden Klemme (3), die klemmend auf ein Gehörknöchelchen aufsetzbar ist, so dass sich das Gehörknöchelchen zwischen den beiden Klemmbacken (9) befindet und die Gehörknöchelchenprothese (1) klemmend an dem Gehörknöchelchen befestigt ist, **dadurch gekennzeichnet, dass** das Kopfelement (2) mehrere gegeneinander bewegliche Kopfteile (4) aufweist und dass ein Kopfteil (4) mit einer Klemmbacke (9) und ein anderes Kopfteil (4) mit einer anderen Klemmbacke (9) so verbunden ist, dass durch eine Bewegung der Kopfteile (4) gegeneinander die Klemmbacken (9) voneinander entfernt oder einander genähert werden können, und dass die Gehörknöchelchenprothese (1) nach Art einer federnden Zange ausgebildet ist, wobei die Kopfteile (4) Zangengriffe oder Teile von Zangengriffen bilden, die bei einer Entfernung voneinander oder einer Annäherung zueinander die Klemmbacken (9) einander nähern oder voneinander entfernen.

2. Gehörknöchelchenprothese nach Anspruch 1, **dadurch gekennzeichnet, dass** die Klemmbacken (9) durch eine Annäherung der Kopfteile (4) voneinander entfernt oder durch eine Entfernung der Kopfteile (4) voneinander einander genähert werden oder umgekehrt.

3. Gehörknöchelchenprothese nach Anspruch 1 oder 2, **dadurch gekennzeichnet, dass** ein verformbarer Steg (10) die Klemmbacken (9) abstandsveränderlich verbindet.

4. Gehörknöchelchenprothese nach einem oder mehreren der Ansprüche 1 bis 3, **dadurch gekennzeichnet, dass** die Klemmbacken (9) elastisch in eine Ausgangslage zurückfedern.

5. Gehörknöchelchenprothese nach einem oder mehreren der vorhergehenden Ansprüche, **dadurch gekennzeichnet, dass** ein Abstand der Klemmbacken (9) voneinander durch plastische Verformung einstellbar ist und/oder dass die Klemme (3) elastisch verformbar ist, so dass ein Abstand der Klemmbacken (9) voneinander durch eine elastische Verformung der Klemme (3) änderbar ist.

6. Gehörknöchelchenprothese nach einem oder mehreren der vorhergehenden Ansprüche, **dadurch gekennzeichnet, dass** die Klemme (3) U-förmig ist, wobei Schenkel des "U" die Klemmbacken (9) bilden.

7. Gehörknöchelchenprothese nach einem oder mehreren der vorhergehenden Ansprüche, **dadurch gekennzeichnet, dass** die Gehörknöchelchenprothese (1) zwei oder mehr deckungsgleiche Klemmen (3) aufweist, die klemmend auf das Gehörknöchelchen aufsetzbar sind.

8. Gehörknöchelchenprothese nach einem oder mehreren der vorhergehenden Ansprüche, **dadurch gekennzeichnet, dass** einander zugewandte Innenflächen oder Innenränder der Klemmbacken (9) eine Wellung, Zacken oder Zähne mit jeweils zumindest einem Tal und zumindest einem Berg aufweisen.

9. Gehörknöchelchenprothese nach Anspruch 8, **dadurch gekennzeichnet, dass** sich ein Abstand gegenüberliegender Täler voneinander von einem freien Ende der Klemmbacken (9) zu einem geschlossenen Ende der Klemme (3) vergrößert.

10. Gehörknöchelchenprothese nach Anspruch 9, **dadurch gekennzeichnet, dass** die Produkte gebildet aus den Abständen gegenüberliegender Täler und dem jeweiligen Abstand dieser Täler vom durch den Steg (10) vorgegebenen Drehpunkt (13) am geschlossenen Ende gleich sind.

11. Gehörknöchelchenprothese nach einem oder mehreren der Ansprüche 8 bis 10, **dadurch gekennzeichnet, dass** in einer Ausgangslage der Gehörknöchelchenprothese (1) sich ein Abstand gegenüberliegender Berge voneinander von einem freien Ende zu einem geschlossenen Ende der Klemme (3) vergrößert.

12. Gehörknöchelchenprothese nach einem oder mehreren der Ansprüche 8 bis 11, **dadurch gekennzeichnet, dass** in einer Ankopplungslage der Gehörknöchelchenprothese (1) sich ein Abstand gegenüberliegender Berge voneinander von einem freien Ende zu einem geschlossenen Ende verkleinert.

13. Gehörknöchelchenprothese nach einem oder mehreren der vorhergehenden Ansprüche, **dadurch gekennzeichnet, dass** die Gehörknöchelchenprothese (1) einstückig ist.

## Claims

1. Auditory ossicle prosthesis, with a head element (2), which is provided for an arrangement on an eardrum, and with a clamp (3) having two clamping jaws (9) that can be clamped to an auditory ossicle, so that the auditory ossicle is between the two clamping jaws (9) and the auditory ossicle prosthesis (1) is clamped to the auditory ossicle, **characterized in that** the head element (2) has several head parts (4) that can be moved relative to one another and that one head part (4) with a clamping jaw (9) and another head part (4) with another clamping jaw (9) is connected in such a way that by moving the head parts (4) against one another the clamping jaws (9) can be moved away from one another or brought closer to one another, and that the auditory ossicle prosthesis (1) is designed in the manner of resilient pliers, with the head parts (4) forming pliers handles or parts of pliers handles which, when moving away from one another or approaching one another move the clamping jaws (9) towards or away from one another.

2. Auditory ossicle prosthesis according to claim 1, **characterized in that** the clamping jaws (9) by moving the head parts (4) closer together are moved apart from another or by moving the head parts (4) away from another move closer together or vice versa.

3. Auditory ossicle prosthesis according to claim 1 or 2, **characterized in that** a deformable bar (10) connects the clamping jaws (9) at an adjustable distance.

4. Auditory ossicle prosthesis according to one or more of claims 1 to 3, **characterized in that** the clamping jaws (9) spring back elastically into an initial position.

5. Auditory ossicle prosthesis according to one or more of the preceding claims, **characterized in that** a distance between the clamping jaws (9) can be adjusted by plastic deformation and/or that the clamp (3) is elastically deformable, so that a distance between the clamping jaws (9) can be changed by an elastic deformation of the clamp (3) can.

6. Auditory ossicles prosthesis according to one or more of the preceding claims, **characterized in that** the clamp (3) is U-shaped, with legs of the "U" forming the clamping jaws (9).

7. Auditory ossicle prosthesis according to one or more of the preceding claims, **characterized in that** the auditory ossicle prosthesis (1) comprises two or more congruent clamps (3) which can be clamped onto the auditory ossicle.

8. Auditory ossicle prosthesis according to one or more of the preceding claims, **characterized in that** mutually facing inner surfaces or inner edges of the clamping jaws (9) comprise a corrugation, spikes or teeth each with at least one valley and at least one peak.

9. Auditory ossicle prosthesis according to Claim 8, **characterized in that** a distance between opposite valleys increases from a free end of the clamping jaws (9) to a closed end of the clamp (3).

10. Auditory ossicle prosthesis according to Claim 9, **characterized in that** the products formed from the distances between opposite valleys and the respective distance of these valleys from a pivot point (13) specified by the bar (10) are the same at the closed end.

11. Auditory ossicle prosthesis according to one or more of Claims 8 to 10, **characterized in that** in an initial position of the auditory ossicle prosthesis (1) a distance between opposite peaks increases from a free end to a closed end of the clamp (3).

12. Auditory ossicle prosthesis according to one or more of Claims 8 to 11, **characterized in that** in a coupling position of the auditory ossicle prosthesis (1), a distance between opposing peaks decreases from a free end to a closed end.

13. Auditory ossicle prosthesis according to one or more of the preceding claims, **characterized in that** the auditory ossicle prosthesis (1) is in one piece.

## Revendications

1. Prothèse d'osselet, comportant un élément de tête (2) qui est prévu pour être disposé sur un tympan, et comportant un dispositif de serrage (3) présentant deux mâchoires de serrage (9), lequel dispositif de serrage peut être placé par serrage sur un osselet de sorte que l'osselet se trouve entre les deux mâchoires de serrage (9) et que la prothèse d'osselet (1) est fixée par serrage sur l'osselet, **caractérisée en ce que** l'élément de tête (2) présente plusieurs parties de tête (4) mobiles l'une par rapport à l'autre **et en ce qu'**une partie de tête (4) est reliée à une mâchoire de serrage (9) et une autre partie de tête (4) est reliée à une autre mâchoire de serrage (9) de sorte que, par un mouvement des parties de tête (4) l'une par rapport à l'autre, les mâchoires de serrage (9) peuvent être éloignées ou rapprochées l'une de l'autre, **et en ce que** la prothèse d'osselet (1) est réalisée à la manière d'une pince élastique, dans laquelle les parties de tête (4) forment des poignées de pince ou des parties de poignées de pince qui, lorsqu'elles sont éloignées ou rapprochées l'une de l'autre, rapprochent ou éloignent les mâchoires de serrage (9) l'une de l'autre.

2. Prothèse d'osselet selon la revendication 1, **caractérisée en ce que** les mâchoires de serrage (9) sont éloignées l'une de l'autre par un rapprochement des parties de tête (4) ou sont rapprochées l'une de l'autre par un éloignement des parties de tête (4) ou inversement.

3. Prothèse d'osselet selon la revendication 1 ou 2, **caractérisée en ce qu'**une barrette (10) déformable relie les mâchoires de serrage (9) en modifiant leur écartement.

4. Prothèse d'osselet selon l'une ou plusieurs des revendications 1 à 3, **caractérisée en ce que** les mâchoires de serrage (9) reviennent élastiquement dans une position initiale.

5. Prothèse d'osselet selon l'une ou plusieurs des revendications précédentes, **caractérisée en ce qu'**un écartement entre l'une et l'autre des mâchoires de serrage (9) est réglable par déformation plastique **et/ou en ce que** le dispositif de serrage (3) est élastiquement déformable de sorte qu'un écartement entre l'une et l'autre des mâchoires de serrage (9) est modifiable par une déformation élastique du dispositif de serrage (3).

6. Prothèse d'osselet selon l'une ou plusieurs des revendications précédentes, **caractérisée en ce que** le dispositif de serrage (3) est en forme de U, dans laquelle les branches du « U » forment les mâchoires de serrage (9).

7. Prothèse d'osselet selon l'une ou plusieurs des revendications précédentes, **caractérisée en ce que** la prothèse d'osselet (1) présente deux dispositifs de serrage (3) ou plus coïncidents qui peuvent être placés par serrage sur l'osselet.

8. Prothèse d'osselet selon l'une ou plusieurs des revendications précédentes, **caractérisée en ce que** les surfaces internes ou les bords internes des mâchoires de serrage (9) qui se font face présentent une ondulation, des dentelures ou des dents comportant respectivement au moins un creux et au moins un sommet.

9. Prothèse d'osselet selon la revendication 8, **caractérisée en ce qu'**un écartement entre des creux opposés les uns aux autres augmente d'une extrémité libre des mâchoires de serrage (9) à une extrémité fermée du dispositif de serrage (3).

10. Prothèse d'osselet selon la revendication 9, **caractérisée en ce que** les produits formés par les écartements entre des creux opposés et l'écartement respectif desdits creux par rapport au point de rotation (13) défini par la barrette (10) sont égaux au niveau de l'extrémité fermée.

11. Prothèse d'osselet selon l'une ou plusieurs des revendications 8 à 10, **caractérisée en ce que,** dans une position initiale de la prothèse d'osselet (1), un écartement entre des sommets opposés les uns aux autres augmente d'une extrémité libre à une extrémité fermée du dispositif de serrage (3).

12. Prothèse d'osselet selon l'une ou plusieurs des revendications 8 à 11, **caractérisée en ce que,** dans une position d'accouplement de la prothèse d'osselet (1), un écartement entre des sommets opposés les uns aux autres diminue d'une extrémité libre à une extrémité fermée.

13. Prothèse d'osselet selon l'une ou plusieurs des revendications précédentes, **caractérisée en ce que** la prothèse d'osselet (1) est en une seule pièce.
